(19) Europäisches Patentamt
European Patent Office
Office européen des brevets

(11) EP 2 724 733 A1

(12) EUROPEAN PATENT APPLICATION
published in accordance with Art. 153(4) EPC

(43) Date of publication:
**30.04.2014 Bulletin 2014/18**

(21) Application number: **12803272.9**

(22) Date of filing: **22.06.2012**

(51) Int Cl.:
***A61L 31/00*** *(2006.01)*
***A61K 31/80*** *(2006.01)*
***A61K 47/34*** *(2006.01)*
***A61K 47/48*** *(2006.01)*
***A61M 1/18*** *(2006.01)*
***A61P 7/02*** *(2006.01)*
***A61K 38/55*** *(2006.01)*

(86) International application number:
**PCT/JP2012/065947**

(87) International publication number:
**WO 2012/176862 (27.12.2012 Gazette 2012/52)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.06.2011 JP 2011139268**

(71) Applicant: **Toray Industries, Inc.**
**Tokyo, 103-8666 (JP)**

(72) Inventors:
- **TANAHASHI, Kazuhiro**
  **Otsu-shi, Shiga 5208558 (JP)**
- **SAKAGUCHI, Hirokazu**
  **Otsu-shi, Shiga 5208558 (JP)**
- **SAKAGUCHI, Yuka**
  **Otsu-shi, Shiga 5208558 (JP)**

(74) Representative: **Kador & Partner**
**Corneliusstrasse 15**
**80469 München (DE)**

# (54) HYDROPHOBIC POLYMER COMPOUND HAVING ANTICOAGULANT EFFECT

(57) The present invention provides a hydrophobic polymer compound capable of inhibiting the blood coagulation reactions in both the primary hemostasis stage involving platelets and the coagulation thrombus formation stage involving blood coagulation factors, which hydrophobic polymer compound can be firmly immobilized on the surface of a medical device or medical material in a state where the compound retains its anticoagulant activity. The present invention provides a hydrophobic polymer compound in which a polymer compound inhibiting platelet adhesion is bound with a compound inhibiting blood coagulation reaction.

EP 2 724 733 A1

**Description**

TECHNICAL FIELD

The present invention relates to a hydrophobic polymer compound having an anticoagulant effect.

BACKGROUND ART

A blood coagulation reaction required for coagulating blood is extremely complex reaction that involves a variety of blood coagulation factors and it is believed that the primary hemostasis stage where platelets are involved and the coagulation thrombus formation stage where blood coagulation factors such as thrombin are involved to stabilize and strengthen fibrin are particularly important.

The blood coagulation reaction is indispensable for stopping bleeding caused by injury or the like; however, in cases where the blood coagulation reaction proceeds during artificial dialysis due to contact between the blood and a medical device or medical material such as an extracorporeal circulation circuit, there are risks that formation of coagulation thrombus increases the circulation pressure and causes vascular occlusion.

As a way of reducing these risks, there is known a method of preventing blood coagulation by preliminarily administering heparin, an anticoagulant, to the patient who is to receive artificial dialysis. However, this method presents a number of problems in that, for example, administration of heparin in an excess amount has side effects, the control of the dosage is complicated and the method cannot be applied to those patients who have bleeding tendency.

Recently, in order to avoid these problems, it has been reportedly attempted to inhibit blood coagulation during treatment by immobilizing a heparin-containing compound having an anticoagulant effect onto the surface of a medical device or medical material such as a blood circuit (Patent Documents 1 to 9).

PRIOR ART REFERENCES

PATENT DOCUMENTS

[Patent Document 1] Japanese Translated PCT Patent Application Laid-open No. 2003-507082
[Patent Document 2] Japanese Patent Application Laid-Open Publication No. 2001-213984
[Patent Document 3] Japanese Translated PCT Patent Application Laid-open No. 2004-525888
[Patent Document 4] Japanese Patent Application Laid-Open Publication No. 2006-291193
[Patent Document 5] WO 08/032758
[Patent Document 6] Japanese Patent Application Laid-Open Publication No. 2009-225824
[Patent Document 7] Japanese Patent Application Laid-Open Publication No. 2010-082067 A
[Patent Document 8] Japanese Patent Application Laid-Open Publication No. 2007-181691
[Patent Document 9] Japanese Patent Application Laid-Open Publication No. 2007-181692

SUMMARY OF THE INVENTION

PROBLEMS TO BE SOLVED BY THE INVENTION

However, at present, as a compound having an anticoagulant effect to be immobilized on the surface of a medical device or medial material such as a blood circuit, a specific compound which is capable of inhibiting the blood coagulation reactions in both the primary hemostasis stage where platelets are involved and the coagulation thrombus formation stage where blood coagulation factors are involved is yet to be developed. Furthermore, even if it is tried to immobilize a conventional anticoagulant compound onto the surface of a medical device or medical material, it is difficult to achieve immobilization in such a state where the compound retains sufficient anticoagulant activity and, even when such immobilization is successfully attained, there is still a problem that the immobilized compound is detached from the medical device or medical material and elutes into the blood during treatment. Moreover, in cases where a plurality of compounds are used for inhibiting the blood coagulation reactions in both the primary hemostasis stage involving platelets and the coagulation thrombus formation stage involving blood coagulation factors, it is necessary to regulate the competitive adsorption between the compounds and to control the immobilization ratio and these operations are extremely complicated.

In view of the above, an object of the present invention is to provide a hydrophobic polymer compound capable of inhibiting the blood coagulation reactions in both the primary hemostasis stage involving platelets and the coagulation

thrombus formation stage involving blood coagulation factors, which hydrophobic polymer compound can be firmly immobilized on the surface of a medical device or medical material in a state where the compound retains its anticoagulant activity.

MEANS FOR SOLVING THE PROBLEMS

In order to solve the above-described problems, the present inventors intensively studied to discover that a hydrophobic polymer compound in which a compound inhibiting blood coagulation reaction is bound to a polymer compound inhibiting platelet adhesion shows a prominent anticoagulant effect and can be firmly immobilized onto the surface of a medical device or medical material.

That is, the present invention provides a hydrophobic polymer compound in which a polymer compound inhibiting platelet adhesion is bound with a compound inhibiting blood coagulation reaction.

It is preferred that the above-described polymer compound inhibiting platelet adhesion be a copolymer composed of a hydrophobic polymer and a hydrophilic polymer and adsorb to polymethyl methacrylate in an amount of not less than 0.1 pg/mm$^2$. The polymer compound inhibiting platelet adhesion is more preferably a copolymer of monomers selected from the group consisting of ethylene glycol, vinyl acetate, vinyl chloride, styrene, acrylic acid, acrylate, alkyl acrylate, hydroxyalkyl acrylate, methacrylic acid, methacrylate, alkyl methacrylate, hydroxyalkyl methacrylate, acrylamide, N-alkylamide, N,N-dialkylacrylamide, methacrylamide, N-alkylmethacrylamide, N,N-dialkylmethacrylamide, acrylonitrile, vinyl pyrrolidone, propylene glycol, vinyl alcohol, ethylene, propylene, ethyleneimine, allylamine, vinylamine and siloxane, or a block copolymer composed of the above-described copolymer of monomers and a polymer selected from the group consisting of polyester, polyamide, polyurethane, polysulfone, polyether sulfone, polycarbonate, polyphenylene sulfide and polyether ether ketone, still more preferably a polyether-modified silicone.

It is preferred that the above-described compound inhibiting blood coagulation reaction have an antithrombin activity. The compound inhibiting blood coagulation reaction is more preferably a compound represented by the following Formula (I), still more preferably (2R,4R)-4-methyl-1-((2S)-2-{[(3RS)-3-methyl-1,2,3,4-tetrahydroquinolin-8-yl]sulfonyl} amino-5-guanidinopentanoyl)piperidine-2-carboxylic acid.

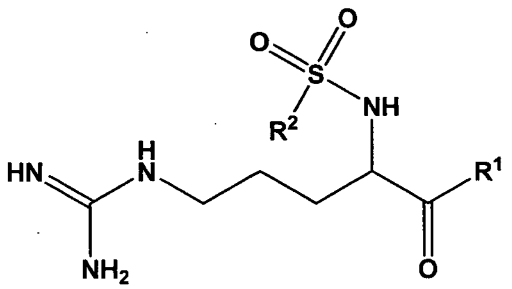

(I)

[wherein, R$^1$ represents a (2R,4R)-4-alkyl-2-carboxypiperidino group; and R$^2$ represents a phenyl group or a fused polycyclic compound group, the fused polycyclic compound group being optionally substituted with a lower alkyl group, a lower alkoxy group or an amino group substituted with a lower alkyl group].

Further, the present invention provides a surface treatment agent of a medical device or medical material, which comprises the above-described hydrophobic polymer compound and has an anticoagulant effect.

Still further, the present invention provides a medical device or a medical material which is treated with the above-described surface treatment agent.

EFFECTS OF THE INVENTION

According to the present invention, the blood coagulation reactions in both the primary hemostasis stage involving platelets and the coagulation thrombus formation stage involving blood coagulation factors can be markedly inhibited and the hydrophobic polymer compound can be firmly immobilized on the surface of a medical device or medical material in a state where the compound retains its anticoagulant activity. Moreover, the hydrophobic polymer compound according to the present invention can be utilized as a surface treatment agent which imparts an anticoagulant effect to a medical device or medical material.

BRIEF DESCRIPTION OF THE DRAWINGS

[Fig. 1] Fig. 1 is a schematic view showing a mini-module prepared in an example.

[Fig. 2] Fig. 2 is a schematic view showing a closed circuit used in an in vitro blood circulation test.

[Fig. 3] Fig. 3 is a schematic view showing a human plasma circulation circuit used in the measurement of the amount of eluted hydrophobic polymer compound.

MODE FOR CARRYING OUT THE INVENTION

The terms used herein are defined as follows unless otherwise specified.

The "hydrophobic polymer compound" according to the present invention is characterized by comprising a polymer compound inhibiting platelet adhesion and a compound inhibiting blood coagulation reaction that are bound to each other. The term "hydrophobic" used herein means that the compound is insoluble to water and does not interact with water molecule by electrostatic interaction or hydrogen bond. Examples of the "hydrophobic polymer compound" according to the present invention include hydrophobic polymer compounds in which a copolymer of monomers selected from the group consisting of ethylene glycol, vinyl acetate, vinyl chloride, styrene, acrylic acid, acrylate, alkyl acrylate, hydroxyalkyl acrylate, methacrylic acid, methacrylate, alkyl methacrylate, hydroxyalkyl methacrylate, acrylamide, N-alkylamide, N,N-dialkylacrylamide, methacrylamide, N-alkylmethacrylamide, N,N-dialkylmethacrylamide, acrylonitrile, vinyl pyrrolidone, propylene glycol, vinyl alcohol, ethylene, propylene, ethyleneimine, allylamine, vinylamine and siloxane, or a block copolymer composed of the above-described copolymer and a polymer selected from the group consisting of polyester, polyamide, polyurethane, polysulfone, polyether sulfone, polycarbonate, polyphenylene sulfide and polyether ether ketone, is bound with a compound represented by the following Formula (I).

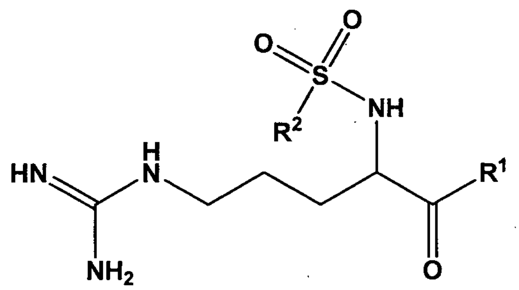
(I)

[wherein, $R^1$ represents a (2R,4R)-4-alkyl-2-carboxypiperidino group; and $R^2$ represents a phenyl group or a fused polycyclic compound group, the fused polycyclic compound group being optionally substituted with a lower alkyl group, a lower alkoxy group or an amino group substituted with a lower alkyl group].

The "polymer compound inhibiting platelet adhesion" means a polymer compound having a number-average molecular weight of not less than 1,000, which has blood compatibility and is capable of inhibiting adhesion of platelets to the surface of a substrate or material when allowed to exist on the surface of a medical device or medical material.

Examples of the "polymer compound inhibiting platelet adhesion" include polyvinyl alcohol; polyvinyl pyrrolidone; polyethylene glycol; polypropylene glycol; polymer compounds composed of highly hydrophobic polysiloxane and polyether; polyethyleneimine; polyallylamine; polyvinylamine; polyvinyl acetate; polyacrylic acid; polyacrylamide; polyhydroxyethyl methacrylate; and block copolymers composed of a monomer of these polymer compounds and a copolymer of other monomers or other polymer. From the standpoint of binding thereto a compound inhibiting blood coagulation reaction, it is preferred that the "polymer compound inhibiting platelet adhesion" have an amino group, a carboxyl group, a hydroxyl group, an epoxy group, an isocyanate group, an isothiocyanate group or a mercapto group. For adsorption to the surface of a medical device or medical material, the "polymer compound inhibiting platelet adhesion" is more preferably a copolymer composed of a hydrophobic polymer and a hydrophilic polymer, still more preferably a polymer compound composed of highly hydrophobic polysiloxane and polyether, a partially saponified polyvinyl alcohol or a copolymer of vinyl pyrrolidone and vinyl acetate.

Examples of the "polymer compound composed of highly hydrophobic polysiloxane and polyether" include copolymers, polymer complexes and polymer blends of highly hydrophobic polysiloxane and polyether. The copolymer of highly hydrophobic polysiloxane and polyether is composed of polyether units and polysiloxane units and the copolymerization mode thereof may be any of a random copolymer, a block copolymer and a graft copolymer. Thereamong, a highly hydrophobic polyether-modified silicone is preferred.

Examples of the "polyether" include structures originated from polyethylene oxide or polypropylene oxide. It is noted here that the term "polyether" refers to a structure represented by the Formula (II) (wherein, $R^3$ represents an alkyl group having not more than 6 carbon atoms) and the term "structure originated from polypropylene glycol", which is one example of polyether, refers to a structure represented by the Formula (III).

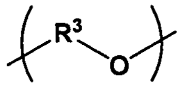
(II)

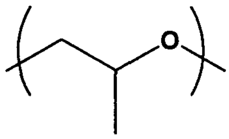
(III)

The term "polyether-modified silicone" refers to a silicone in which polyether units are bound as side chains of the silicone chain, and the "polyether-modified silicone" may be a polyether-modified silicone which is further amino-modified or carboxy-modified with binding of amino groups or carboxyl groups to the side chain.

In cases where the polymer compound inhibiting platelet adhesion is a partially-saponified polyvinyl alcohol, the saponification degree thereof is, from the viewpoint of attaining suitable ease of handling or hydrophobicity, preferably 10 to less than 65 mol%, more preferably 15 to 60 mol%, still more preferably 15 to 55 mol%. The term "saponification degree" used herein refers to a numerical value calculated by the following Equation 1.

$$\text{Saponification degree} = m/(n + m) \times 100 \qquad \text{Equation 1}$$

m: the number of structures represented by the Formula (IV) in polyvinyl alcohol
n: the number of structures represented by the Formula (V) in polyvinyl alcohol

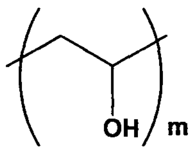
(IV)

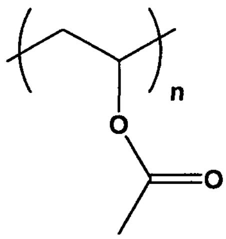
(V)

In cases where the polymer compound inhibiting platelet adhesion is a copolymer of vinyl pyrrolidone and vinyl acetate, from the viewpoint of attaining suitable ease of handling, adsorption to a substrate or hydrophobicity, the amount of vinylpyrrolidone units is preferably less than 50 unit mol%, more preferably 20 to less than 49.9 unit mol%. It is noted here that the proportion of the vinylpyrrolidone units in the copolymer of vinyl pyrrolidone and vinyl acetate (unit mol%) can be determined by $^1$H-NMR measurement (solvent: $CDCl_3$) of the copolymer.

The adsorption amount of the polymer compound inhibiting platelet adhesion to a substrate such as a medical device or a medical material is preferably not less than 0.1 pg/mm$^2$, more preferably not less than 1 pg/mm$^2$, still more preferably not less than 10 pg/mm$^2$.

The above-described adsorption amount is measured by the following method. First, an untreated sensor chip (Sensor Chip Au; GE Healthcare) is pre-treated (with 25°C distilled water, at flow rate of 20 μl/min, for 10 minutes) using a surface plasmon resonance apparatus (hereinafter, referred to as "SPR") (BIACORE 3000; GE Healthcare) and the signal value (RU: resonance unit) is measured.

The "substrate", that is, an adsorbent material, is dissolved in a solvent to prepare a 0.5%-by-weight solution of adsorbent material. One drop of the thus obtained solution of adsorbent material is dropped onto the center of the gold film part of a pre-treated sensor chip installed in a spin coater, which is rotated immediately thereafter at 3,000 rpm for 1 minute at room temperature to coat the sensor chip with the adsorbent material.

After confirming that no droplet is present on the sensor chip, the sensor chip is washed with distilled water using SPR (25°C, flow rate: 20 μl/min, 10 minutes). Then, the resulting sensor chip is further washed three times with 0.025%-by-weight Triton-X100 solution (25°C, flow rate: 20 μl/min, 1 minute) and the signal value is measured at 10 minutes after the completion of the washing.

Of the sensor chips obtained as described above, one which has a difference in the signal value before and after the spin coating in the range of 3,000 to 8,000 is selected. After being washed with distilled water (25°C, flow rate: 20 μl/min, 10 minutes), the selected sensor chip is further washed three times with 0.025%-by-weight Triton-X100 solution (25°C, flow rate: 20 μl/min, 1 minute).

Ten minutes after the completion of the washing, a methanol solution of a hydrophobic polymer compound to be adsorbed to a substrate (concentration: 100 μg/ml) is injected (25°C, flow rate: 20 μl/min, 1 minute) and then washed with distilled water (25°C, flow rate: 20 μl/min, 3 minutes). The difference between the signal value immediately before the start of the injection (hereinafter, referred to as "signal value A") and the signal value at 3 minutes after the completion of the injection (hereinafter, referred to as "signal value B") is determined and converted in terms of 1 RU = 1 pg/$mm^2$.

Subsequently, the sensor chip is washed with distilled water (25°C, flow rate: 20 μl/min, 2 minutes) and further washed three times with 0.025%-by-weight Triton-X100 solution (25°C, flow rate: 20 μl/min, 1 minute), and the methanol solution of the hydrophobic polymer compound to be adsorbed (concentration: 100 μg/ml) is injected again (25°C, flow rate: 20 μl/min, 1 minute). Thereafter, the same operations are repeated for a total 5 times to determine the signal difference (difference between the signal value A and the signal value B) for each time and the average thereof is taken as the amount of the polymer compound inhibiting platelet adhesion adsorbed to the substrate".

The "compound inhibiting blood coagulation reaction" refers to a compound having an anti-blood coagulation capacity such as antithrombin activity. More specifically, the "compound inhibiting blood coagulation reaction" refers to a compound which, when added to blood at a concentration of 10 μg/mL, prolongs the prothrombin time by 30% or more as compared to a blank blood.

The "prothrombin time" is measured by the method described in a known literature (Masamitsu Kanai et al., "Clinical Test Handbook, vol. 30", Kanehara & Co., Ltd., 1993, p.416-418). Specifically, 1 volume of 3.2% sodium citrate and 9 volumes of blood are mixed and 0.1 mL aliquot of citrated plasma is recovered in a small test tube (inner diameter = 8 mm, length = 7.5 cm). Then, after warming the resulting mixture by placing the small test tube in a 37°C thermostat bath for 3 minutes, 0.2 mL of a tissue thromboplastin-calcium reagent kept at 37°C is further added to the mixture. After gently shaking the small test tube, the small test tube is left to stand in a tilted condition to allow fibrin to precipitate. Here, the time required for fibrin to precipitate after the addition of the issue thromboplastin-calcium reagent is measured and defined as "prothrombin time".

Examples of the "compound inhibiting blood coagulation reaction" include heparin, nafamostat mesilate, sodium citrate, sodium oxalate, α1-antitrypsin, α2-macroglobulin, C1 inhibitors, thrombomodulin, protein C, compounds having a guanidino structure, prostaglandin, hirudin, Xa inhibitors, tissue factor inhibitors, urokinase and antithrombin; however, the "compound inhibiting blood coagulation reaction" is preferably a compound having an antithrombin activity.

The term "compound having an antithrombin activity" means a compound having a high binding affinity to thrombin.

Examples of an index for evaluating the antithrombin activity of a compound include inhibition constant (hereinafter, referred to as "Ki") which is determined from a Lineweaver-Burk plot based on the absorbance value of a test solution. A smaller Ki indicates a higher binding affinity to thrombin, that is, a higher antithrombin activity.

Examples of the "compound having an antithrombin activity" include compounds having a guanidino structure, and the "compound having an antithrombin activity" is preferably (2R,4R)-4-methyl-1-((2S)-2-{[(3RS)-3-methyl-1,2,3,4-tetrahydroquinolin-8-yl]sulfonyl}amino-5-guanidinopentanoyl)piperidine-2-carboxylic acid (hereinafter, referred to as "argatroban"). Argatroban synthesized in 1978 is a medicinal compound having a selective antithrombin activity of arginine derivatives.

Further, the surface treatment agent of a medical device or medical material according to the present invention is characterized by comprising the above-described hydrophobic polymer compound and having an anticoagulant effect.

Examples of the "medical device or medical material" include implantable artificial organs; synthetic blood vessels; catheters; stents; blood bags; contact lenses; intraocular lenses; surgical auxiliary instruments; and separation membranes and adsorbents that are integrated in biological component separation modules and blood purification modules.

Examples of a method of treating the surface of a medical device or medical material with the above-described surface treatment agent, that is, a method of immobilizing the above-described hydrophobic polymer compound, which is an active ingredient of the surface treatment agent, onto the surface of a medical device or medical material, include a method in which the above-described surface treatment agent is brought into contact with a medical device or medical material and radiation is then irradiated thereto. As for the type of the radiation, an electron beam and γ-ray are preferred.

Examples of the material of the "medical device or medical material" include cellulose, cellulose acetate, polycarbonate, polysulfone, polyether sulfone, polymethacrylate such as polymethyl methacrylate (hereinafter, referred to

as "PMMA"), polyacrylate, polyamide, polyvinylidene fluoride, polyvinyl chloride, polyacrylonitrile, polyester, polyurethane, polystyrene, polyethylene, polypropylene, polymethylpentene, polyether ether ketone, silicon and polyimide.

EXAMPLES

The present invention will now be described in more detail by way of examples thereof; however, the present invention is not restricted to the following examples.

(Example 1: Binding between Polyether-modified Silicone and Argatroban)

In 50 mL of anhydrous dimethylformamide (hereinafter, referred to as "anhydrous DMF"), 15.4 g of amino-modified silicone (KF-865; Shin-Etsu Chemical Co., Ltd.) was dissolved to prepare an amino-modified silicone/anhydrous DMF solution. Also, 0.3 g of 1-ethyl-3-(3-dimethylaminopropyl)carbodiimide (hereinafter, referred to as "EDC") was dissolved in 5 mL of anhydrous DMF to prepare an EDC/anhydrous DMF solution. Further, 0.3 g of 4-hydroxybenzotriazole (hereinafter, referred to as "HOBt") was dissolved in 5 mL of anhydrous DMF to prepare a HOBt/anhydrous DMF solution.

While ice-cooling the whole amount of the thus prepared amino-modified silicone/anhydrous DMF solution, the whole amounts of the EDC/anhydrous DMF solution and the HOBt/anhydrous DMF solution were added and 2.1 g of N-hydroxysuccinimidized polyethylene glycol (SUNBRIGHT ME-020AS; NOF Corporation) was further added. The resulting mixture was allowed to react at room temperature for 3 days. Then, the thus obtained reaction solution was placed in a dialysis tube (SPECTRA/POR RC, Pore 6, MWCO = 1,000) and dialyzed for 3 days in distilled water having an amount of more than 10 times the volume of the reaction solution while replacing the distilled water as appropriate. The thus dialyzed reaction solution was filtered and the resulting insoluble matter was dried overnight in a vacuum dryer to obtain a polyether-modified silicone (hereinafter, referred to as "polyether-modified silicone A").

After placing 5 mmol of argatroban into a recovery flask and dissolving the argatroban with an addition of 10 mL of anhydrous DMF, 10 mL of 4N hydrochloric acid/1,4-dioxane (Toyo Kasei Co., Ltd.) was added thereto dropwise while ice-cooling the recovery flask and the resulting mixture was stirred for 1 hour. Then, the solvent was distilled off using a rotary evaporator. Further, the resultant was dried overnight in a vacuum dryer and 25 mL of anhydrous DMF was added thereto to prepare an argatroban hydrochloride/anhydrous DMF solution.

The thus prepared argatroban hydrochloride/anhydrous DMF solution was placed in a two-necked flask in the amount shown in Table 1 and, while ice-cooling the flask, the EDC/anhydrous DMF solution and the HOBt/anhydrous DMF solution were added. The polyether-modified silicone A was further added thereto and the resulting mixture was allowed to react at room temperature for 3 days. Then, the thus obtained reaction solution was placed in a dialysis tube (SPECTRA/POR RC, Pore 6, MWCO = 1,000) and dialyzed for 3 days in distilled water having an amount of more than 10 times the volume of the reaction solution while replacing the distilled water as appropriate. The thus dialyzed reaction solution was filtered and the resulting insoluble matter was dried overnight in a vacuum dryer to obtain a hydrophobic polymer compound (hereinafter, referred to as "Example 1 Compound").

(Measurement of Antithrombin activity of Example 1 Compound)

For the measurement, ECA-T Kit (HaemoSys GmbH) was used. To 100 μL of Example 1 Compound/methanol solution prepared by dissolving 0.5 g of the Example 1 Compound in 1 mL of methanol, 900 μL of distilled water was added to prepare Example 1 Compound dispersion. This Example 1 Compound dispersion was recovered in an amount of 30 μL and mixed with 100 μL of ECA prothrombin buffer and 25 μL of ECA-T substrate. After incubating the resulting mixture at 37°C for 60 seconds, the mixture was set in an apparatus (COATRON M1 (code 80 800 000); TECO Medical Instruments, Production + Trading GmbH) and 50 μL of ECA ecarin reagent was further added thereto to carry out the measurement.

In place of the above-described Example 1 Compound dispersion, a mixture of 20 μL of an argatroban solution prepared to have an arbitrary concentration using an ethanol/hydrochloric acid (volume ratio: 4/1) mixed solvent and 80 μL of human plasma and a mixture of 20 μL of blank (distilled water) and 80 μL of human plasma were each subjected to the measurement using the ECA-T kit, and a calibration curve was prepared from the results thereof. The concentration of 1,450 ppm by weight in terms of argatroban of the Example 1 Compound dispersion, which was calculated based on the calibration curve, was defined as the value indicating the antithrombin activity of the Example 1 Compound dispersion.

(Examples 2 to 6)

Example Compounds 2 to 6 were obtained and their antithrombin capacities were measured in the same manner as in Example 1 except that the molar ratios of EDC, HOBt, KF-861 and SUNBRIGHT ME-020AS to argatroban hydrochloride and the volume ratio of anhydrous DMF to the polyether-modified silicone A were changed. The molar ratios

of EDC, HOBt, KF-861 and SUNBRIGHT ME-020AS to argatroban hydrochloride and the results of measuring the antithrombin capacities of the Example Compounds 2 to 6 are shown in Table 1.

[Table 1]

| Compound | Molar ratio with respect to 1.00 mol of argatroban hydrochloride | | | | Volume ratio of anhydrous DMF with respect to 1 volume of polyether-modified silicone | Concentration in terms of argatroban (ppm by weight) |
|---|---|---|---|---|---|---|
| | EDC | HOBt | KF-861 | ME-020AS | | |
| Example 1 | 1 | 1 | 0.3 | 0.3 | - | 850 |
| Example 2 | 1 | 1 | 0.2 | 0.2 | - | 660 |
| Example 3 | 1 | 1 | 0.1 | 0.1 | - | 500 |
| Example 4 | 1 | 1 | 0.5 | 0.5 | 4 | 440 |
| Example 5 | 1 | 1 | 0.2 | 0.2 | 4 | 480 |
| Example 6 | 1 | 1 | 0.1 | 0.1 | 4 | 720 |

Here, the antithrombin activity of the polyether-modified silicone A described in Example 1 was also measured; however, the measured value was not different from that of the blank (distilled water), so that it was confirmed that the polyether-modified silicone A itself has no antithrombin activity.

(Measurement of Thrombin Inhibition Constant of Example 1 Compound)

An aqueous bovine thrombin solution was prepared by dissolving 10,000 U of a bovine thrombin solution (ILS Inc.) into 1 mL of physiological saline.

An aqueous S-2238 stock solution was prepared by dissolving 25 mg of S-2238 stock solution (Sekisui Medical Co., Ltd.) into 40 mL of distilled water.

The above-described aqueous bovine thrombin solution, aqueous S-2238 stock solution and Example 1 Compound/methanol solution were each diluted with a dilution buffer (0.05M Tris, 0.1M NaCl, 1 mg/mL bovine serum albumin (BSA), pH = 7.4).

To a 96-well plate, 100 μL of the thus diluted aqueous S-2238 stock solution and 50 μL of the thus diluted Example 1] Compound/methanol solution were aliquoted, and the resulting plate was sealed and then warmed for 30 minutes in a thermostat dryer set at 37°C. Thereafter, 50 μL of the diluted aqueous bovine thrombin solution, which had been warmed at 37°C for 30 minutes, was further aliquoted and the absorbance of the resultant was measured immediately thereafter using a microplate reader (measurement wavelength = 405 nm, reference wavelength = 595 nm).

Immediately after the completion of the first absorbance measurement, the second absorbance measurement was performed. The third and subsequent absorbance measurements were performed at 4, 6, 8, 10, 12, 14, 16, 18 and 20 minutes after the addition of the diluted aqueous bovine thrombin solution, respectively. Ki was calculated from a Lineweaver-Burk plot of the thus obtained absorbance values. The Ki of the Example 1 Compound was 36 nM.

Here, the Ki was also calculated for the polyether-modified silicone A having no antithrombin activity; however, as expected, it was the same as the Ki of the blank.

Further, when the Ki of argatroban was calculated in the same manner, the Ki was found to be 46 nM, which was 1.3 times higher than that of the Example 1 Compound.

From these results, it is apparent that the above-described hydrophobic polymer compound has an extremely high binding affinity to thrombin and is capable of imparting a medical device or medical material, such as a hollow fiber-type dialyzer, with a prominent antithrombin activity at a level much higher than that of argatroban known to have an antithrombin activity.

(Preparation of PMMA Hollow Fiber Membrane Mini-module)

To 75 parts by weight of dimethyl sulfoxide, 5 parts by weight of isotactic-PMMA and 20 parts by weight of syndiotactic-PMMA were added, and the resulting mixture was stirred at 110°C for 8 hours to obtain a membrane-forming stock solution. The thus obtained membrane-forming stock solution was discharged from an orifice-type coaxial cylindrical mouthpiece and, after allowing the discharged solution to pass through the air for a length of 300 mm, the resulting solution was introduced into a coagulation bath of 100% water to obtain PMMA hollow fibers of 0.2 mm in inner diameter and 0.03 mm in membrane thickness. It is noted here that dry nitrogen was used as the gas injected into the fibers.

In the same manner as in the case of a conventional hollow fiber-type dialyzer, a module case of 10 mm in inner diameter and 120 mm in length, which had two ports communicating to the interior of the hollow fibers (blood ports) and two ports communicating to the outside of the hollow fibers (dialysate ports), was prepared.

Fifty of the above-described PMMA hollow fibers were bundled to form a PMMA hollow fiber membrane and, with attention being paid not to clog the hollow parts of the thus obtained PMMA hollow fiber membrane, both ends of the membrane were fixed to the above-described module case using an epoxy-based potting agent. Then, the PMMA hollow fiber membrane and the inside of the module case were washed with distilled water to obtain a mini-module 6 shown in Fig. 1.

(Immobilization of Example 1 Compound onto PMMA hollow fiber membrane)

Distilled water remaining on the blood-contacting side (the inner side of the PMMA hollow fiber membrane) and the blood non-contacting side (the outer side of the PMMA hollow fiber membrane) of the thus obtained mini-module 6 was removed by blowing compressed air. Then, a propylene glycol solution of the Example 1 Compound having a concentration of 4,000 ppm by weight in terms of argatroban was prepared a filling solution.

Using a syringe, 400 $\mu$L of the thus obtained filling solution was filled only in the blood-contacting side of the mini-module 6. Then, after removing the filling solution by blowing compressed air, all of the blood ports 1a and 1b and the dialysate ports 2a and 2b of the mini-module 6 were tightly capped and the mini-module 6 was irradiated with $\gamma$-ray at an absorbed dose of 25 kGy.

The PMMA hollow fiber membrane 4 and the interior of the mini-module 6 were washed by allowing 0.025%-by-weight aqueous polyoxyethylene octylphenyl ether solution to flow therethrough at a flow rate of 10 mL/min for 8 hours using a peristaltic pump 8. Then, the PMMA hollow fiber membrane 4 and the interior of the mini-module 6 were further washed by allowing methanol, distilled water and physiological saline to flow therethrough at a flow rate of 10 mL/min for 30 minutes each, thereby obtaining a mini-module in which the Example 1 Compound (hereinafter, referred to as "Example 1 Mini-module") was immobilized.

Meanwhile, a mini-module in which the polyether-modified silicone A was immobilized (hereinafter, referred to as "Comparative Example 1 Mini-module") was obtained by the same operations as described above except that the polyether-modified silicone A was used in place of the Example 1 Compound having concentration of 4,000 ppm by weight in terms of argatroban.

(In Vitro Blood Circulation Test)

Blood donated by a volunteer and citric acid were mixed at a volume ratio of 9/1 to obtain a citric acid-supplemented blood. To 1 mL of this citric acid-supplemented blood, 43.6 $\mu$L of calcicol was added as a procoagulant to prepare a test blood.

Silicon tubes 7a and 7b were connected to the Example 1 Mini-module and the peristaltic pump 8 was arranged in the middle of the silicon tube 7b. From the silicon tube 7a connected to the blood port 1 a, the test blood was allowed to flow at a flow rate of 0.9 mL/min for 5 seconds. The test blood discharged from the blood port 1b was discarded via the silicon tube 7b and the bubbles formed inside the PMMA hollow fiber membrane were removed. Then, the silicon tubes 7a and 7b were connected at a connecting part 9 to prepare the closed circuit shown in Fig. 2.

Circulation of the test blood was started at a flow rate of 0.9 mL/min and the duration of circulation sustained until the silicon tube 7a or 7b was detached from the connecting part 9 due to an increase in the internal pressure of the circuit caused by coagulation thrombus formed in the circuit was measured. When the Example 1 Mini-module was used, the duration of circulation was 37 minutes.

A mini-module 6 in which no compound was immobilized on the PMMA hollow fiber membrane (hereinafter, referred to as "Comparative Example 2 Mini-module") was prepared and subjected to the same blood circulation test as described above. The duration of circulation in this case was measured to be 20 minutes, which was not more than 60% of the case where Example 1 Mini-module was used. From these results, it is apparent that the above-described hydrophobic polymer compound is capable of imparting an excellent anticoagulant effect to a medical device or medical material such as a hollow fiber-type dialyzer.

It is noted here that, when the same blood circulation test as described above was performed using the Comparative Example 1 Mini-module, the duration of circulation was measured to be 20 minutes, which was not different from the value obtained by using the Comparative Example 2 Mini-module in which no compound was immobilized on the PMMA hollow fiber membrane.

(Measurement of Eluted Amount of Example 1 Compound)

The silicon tube 7b of 0.8 mm in inner diameter and 520 mm in length was connected to the blood port 1b of

the separately prepared Example 1 Mini-module and the peristaltic pump 8 was arranged in the middle of the silicon tube 7b. To the blood port 1a, the silicon tube of 0.8 mm in inner diameter and 160 mm in length was connected. Then, the other ends of the silicon tubes 7a and 7b were each inserted into a polystyrene round tube 10 (Code: 352054; Becton, Dickinson and Co.) containing 5 mL of human plasma, thereby preparing the circulation circuit shown in Fig. 3.

After allowing the Example 1 Compound to circulate in the human plasma at a flow rate of 0.5 mL/min for 4 hours using the peristaltic pump 8, the concentration of the Example 1 Compound in the human plasma contained in the polystyrene round tube 10 was measured using the ECA-T Kit. However, the concentration of the Example 1 Compound in the human plasma after the circulation was below the detection limit of the ECA-T Kit, so that elution of the Example 1 Compound from the Example 1 Mini-module was not confirmed. This result indicates that the above-described hydrophobic polymer compound can be firmly immobilized onto a medical device or medical material such as a hollow fiber-type dialyzer.

(Evaluation of Adsorbed Amount of Polymer Compound having Platelet Adhesion-inhibiting Capacity)

As a copolymer of vinyl pyrrolidone and vinyl acetate (hereinafter, referred to as "VA-type copolymer"), which is one of the polymer compounds inhibiting platelet adhesion that constitute the above-described hydrophobic polymer compound, VA37 (BASF Corporation) was prepared. Similarly, a partially-saponified polyvinyl alcohol, which is one of the polymer compounds inhibiting platelet adhesion, was also prepared. Furthermore, the polyether-modified silicone A obtained in Example 1 was prepared. The thus prepared VA-type copolymer, partially-saponified polyvinyl alcohol and polyether-modified silicone A were each diluted with methanol to prepare a 10,000 ppm-by-weight methanol solution.

The partially-saponified polyvinyl alcohol was prepared as follows. First, 20 g of vinyl acetate (Wako Pure Chemical Industries, Ltd.) and 20 mg of azobisbutyronitrile (Wako Pure Chemical Industries, Ltd.) were dissolved in anhydrous DMF and the resulting mixture was stirred at 70°C for 5 hours. The thus obtained reaction solution was added to sodium bicarbonate (Wako Pure Chemical Industries, Ltd.) and the precipitated polymer was recovered, washed with water and then dried under reduced pressure. After dissolving 5 g of the thus obtained dry polymer in 15 mL of methanol (Wako Pure Chemical Industries, Ltd.), 0.05 g of sodium hydroxide was added and the resulting mixture was stirred at 60°C to perform hydrolysis reaction. After removing the precipitated polymer by filtration through a glass filter, a residue obtained by removing methanol from the resulting filtrate was dried under reduced pressure to obtain 2.1 g of a partially-saponified polyvinyl alcohol having a saponification degree of 16%. By changing the amount of sodium hydroxide and the time of the hydrolysis reaction, partially-saponified polyvinyl alcohols having various saponification degrees were obtained.

For comparisons, as polymer compounds that are not included in the polymer compound inhibiting platelet adhesion which constitutes the above-described hydrophobic polymer compound, PEG2000, PEG4000, PEG6000 and PEG20000 (all of which are manufactured by Nacalai Tesque, Inc.) as well as PEG methyl ether (PEG-em) and PEG dimethyl ether (PEG-dm) (both of which are manufactured by Sigma-Aldrich Co., LLC.) were prepared. The thus prepared polymer compounds were each diluted with distilled water to prepare a 10,000 ppm-by-weight aqueous solution.

As 0.5%-by-weight solutions of an adsorbent material to which the polymer compound inhibiting platelet adhesion adsorbs, a PMMA (weight-average molecular weight = 93,000; Sigma-Aldrich Co., LLC.)/toluene solution, a polyurethane/dimethylacetamide solution, a polysulfone (UDEL (registered trademark) P-3500; Solvay Specialty Polymers K.K.)/dimethylacetamide solution, a polyvinyl chloride (weight-average molecular weight = 80,000; Sigma-Aldrich Co., LLC.)/tetrahydrofuran solution, a polystyrene (Wako Pure Chemical Industries, Ltd.)/chloroform solution and a polycarbonate (weight-average molecular weight = 20,000; Teijin Ltd.)/chloroform solution were prepared.

The amounts of various polymer compounds inhibiting platelet adhesion that adsorbed to the respective adsorbent materials were measured. The results thereof are shown in Table 2.

[Table 2]

| Signal value B - Signal value A [pg/mm²] | | | | | | | |
|---|---|---|---|---|---|---|---|
| | | Adsorbent material | | | | | |
| | | PMMA | Polysulfone | Polyurethane | Polyvinyl chloride | Polystyrene | Polycarbonate |
| Polymer compound inhibiting platelet adhesion | VA37 | 2,760 | - | - | - | - | - |
| | PVA (16%) | 2,229 | 2,586 | 1,635 | 2,468 | 2,377 | 2,356 |
| | PVA (23%) | 2,360 | 2,642 | 1,611 | 2,330 | 2,168 | 2,346 |
| | PVA (38%) | 2,273 | 2,130 | 1,411 | 1,796 | 1,989 | 1,819 |
| | PVA (52%) | 2,120 | 2,117 | 1,267 | 1,890 | 1,530 | 2,013 |
| | Polyether-modified silicone A | 1,920 | 1,730 | 1,210 | 1,890 | 1,330 | 1,220 |
| | PEG 2000 | 2 | - | - | - | - | - |
| | PEG 4000 | 2 | - | - | - | - | - |
| | PEG 6000 | 5 | - | - | - | - | - |
| | PEG 20000 | 113 | - | - | - | - | - |
| | PEG-me | 5 | - | - | - | - | - |
| | PEG-dm | 67 | - | - | - | - | - |

The degrees in parentheses refer to saponification degree of polyvinyl alcohol.

From the results shown in Table 2, it is apparent that the polymer compound inhibiting platelet adhesion which constitutes the above-described hydrophobic polymer compound is not restricted to polyether-modified silicones and can be firmly adsorbed to a medical device or medical material such as a hollow fiber-type dialyzer.

(Evaluation of Platelet Adhesion-inhibiting Capacity)

The separately prepared module case of the Example 1 Mini-module was cut with an ultrasonic cutter to take out the PMMA hollow fiber membrane (hereinafter, referred to as "Example 1 Hollow Fiber Membrane") on which the Example 1 Compound was immobilized.

A double-sided tape was pasted onto one side of a polyethylene terephthalatemade circular film of 18 mm in diameter and the Example 1 Hollow Fiber Membrane was fixed thereto. Then, the thus fixed PMMA hollow fiber membrane was cut into a semi-cylindrical shape to expose the inner surface thereof. The Example Hollow Fiber Membrane fixed onto the circular film was then placed in a FALCON (registered trademark) cylindrical tube (18 mmφ, No. 2051) cut into a cylindrical shape, and the gap between the cylindrical tube and the circular film was sealed with Parafilm. Thereafter, this cylindrical tube was filled with physiological saline.

Venous blood was collected from a volunteer and immediately thereafter, the venous blood was loaded to a

blood collection tube in which heparin had been collected in advance. The contents were mixed by inversion to prepare heparin-supplemented blood. It is noted here that the heparin-supplemented blood was adjusted to have a heparin concentration of 50 U/mL.

After discarding the physiological saline contained in the above-described cylindrical tube, 1.0 mL of the thus obtained heparin-supplemented blood was loaded and the cylindrical tube was shaken at 37°C for 1 hour. Then, after washing the Example 1 Hollow Fiber Membrane contained in the above-described cylindrical tube with 10 mL of physiological saline, a physiological saline solution containing 2.5% by volume of glutaraldehyde was added to fix the blood component, followed by further washing with distilled water. Thereafter, the circular film on which the Example 1 Hollow Fiber Membrane was fixed was removed from the above-described cylindrical tube and then dried at normal temperature for 12 hours under reduced pressure having an absolute pressure of 0.5 Torr.

After pasting the thus dried circular film, on which the Example 1 Hollow Fiber Membrane was fixed, onto the stage of a scanning electron microscope using a double-sided tape, a platinum/palladium thin film was formed on the surface of the Example Hollow Fiber Membrane by sputtering. The inner surface of the central portion in the longitudinal direction of the Example Hollow Fiber Membrane on which the platinum/palladium thin film was formed was observed under a field emission scanning electron microscope (S800; Hitachi, Ltd.) at a magnification of $\times$ 1,500 and the number of adhered platelets in one field of view ($4.3 \times 10^3\ \mu m^2$) was counted.

The integer of the average number of adhered platelets in 5 different fields of view was defined as the number of adhered platelets (platelets/$4.3 \times 10^3\ \mu m^2$) and the number of adhered platelets on the Example Hollow Fiber Membrane was 15.

Meanwhile, the separately prepared module case of the Comparative Example 2 Mini-module was cut with an ultrasonic cutter to take out the hollow fiber membrane on which no compound was immobilized (hereinafter, referred to as "Comparative Example 2 Hollow Fiber Membrane") and the number of adhered platelets thereon was verified in the same manner. As a result, the number of adhered platelets on the Comparative Example 2 Hollow Fiber Membrane was not less than 100.

From these results, it is apparent that the above-described hydrophobic polymer compound is capable of imparting a prominent platelet adhesion-inhibiting capacity to a medical device or medical material such as a hollow fiber-type dialyzer.

(Measurement of Whole Blood Coagulation Time)

Blood collected from a volunteer and citric acid were mixed at a volume ratio of 9/1 to prepare a citric acid-supplemented blood.

In a cuvette (Non-activated Clotting Test Kit), 18 $\mu$L of physiological saline was placed and 14.8 $\mu$L of calcicol was added thereto, followed by further addition of 342 $\mu$L of the thus obtained citric acid-supplemented blood. The resulting mixture was measured using a Sonoclot blood coagulation/platelet function analyzer (IMI Co., Ltd.) and the measured ACT ONSET value was defined as the whole blood coagulation time. The whole blood coagulation time of the blood collected from the volunteer was 545 seconds.

When the same measurements were carried out using 2, 10 and 20 $\mu$M argatroban solutions (solvent: methanol/hydrochloric acid (volume ratio = 4/1)) in place of physiological saline, the whole blood coagulation time was found to be 520, 734 and 893 seconds, respectively.

When the same measurements were carried out using 1, 2 and 5 $\mu$M Example 1 Compound dispersion in place of physiological saline, the whole blood coagulation time was found to be 590, 780 and 910 seconds, respectively.

(Example 14: Binding between Vinyl Acetate-Vinyl Pyrrolidone Copolymer and Compound 1)

In a screw vial, 14.9 g of tetrahydrofuran, 23.0 g of vinyl acetate, 10.8 g of N-vinylpyrrolidone, 0.028 g of 2-aminoethanethiol and 0.016 g of azobisisobutyronitrile were placed and, after tightly sealing the screw vial, the resulting mixture was ultrasonicated for 10 minutes. Then, the screw vial was once unsealed and the mixture therein was bubbled with argon gas for 10 minutes. After tightly sealing the screw vial again, with stirring of the mixture, the screw vial was immersed in a 60°C hot water bath for 1 hour and then in a 70°C hot water bath for 6 hours, thereby allowing vinyl acetate and vinyl pyrrolidone to be copolymerized. To the thus obtained reaction solution, 80 mL of methanol was added, and the resulting mixture was added to about 5 times amount of ether, followed by removal of the resulting supernatant. After repeating three times the washing operation in which ether was freshly added and the resulting supernatant was removed, the resultant was dried under reduced pressure to obtain a vinyl acetate-vinyl pyrrolidone copolymer. The thus obtained vinyl acetate-vinyl pyrrolidone copolymer was subjected to $^1$H-NMR measurement (solvent: $CDCl_3$) and the amount of vinylpyrrolidone unit was found to be 28.6 unit mol%.

In 20 mL of anhydrous DMF, 4.6 g of the thus obtained vinyl acetate-vinyl pyrrolidone copolymer was dissolved to prepare a vinyl acetate-vinyl pyrrolidone copolymer/anhydrous DMF solution. The entire amount of the thus obtained

vinyl acetate-vinyl pyrrolidone copolymer/anhydrous DMF solution and 0.5 mL of an argatroban hydrochloride/anhydrous DMF solution (0.49M) were placed in a two-necked flask and, while ice-cooling the flask and stirring the solution in the flask, 0.5 mL of EDC/anhydrous DMF solution (1.04M) and 0.5 mL of HOBt/anhydrous DMF solution (1.02M) were added. The resulting mixture was allowed to react for 3 days under a nitrogen atmosphere at room temperature. Then, the thus obtained reaction solution was placed in a dialysis tube (SPECTRA/POR RC, Pore 6, MWCO = 1,000) and dialyzed for 3 days in distilled water having an amount of more than 10 times the volume of the reaction solution while replacing the distilled water as appropriate. The thus dialyzed reaction solution was filtered and, after distilling off the solvent of the filtrate using a rotary evaporator, the resultant was dried overnight in a vacuum dryer to obtain a hydrophobic polymer compound (hereinafter, referred to as "Example 7 Compound").

(Measurement of Antithrombin activity of Example 7 Compound)

The antithrombin activity of an Example 7 Compound/methanol solution (concentration: 20% by weight) was measured in the same manner as the measurement of the antithrombin activity of the Example 1 Compound and the calculated Compound 1-equivalent concentration of 87 ppm of the Example 7 Compound/methanol solution was defined as the value indicating the antithrombin activity of the Example 7 Compound/methanol solution.

From these results, it is apparent that, as compared to argatroban known to have an antithrombin activity, the above-described hydrophobic polymer compound is capable of prolonging the whole blood coagulation time even at an extremely low concentration and imparting an excellent anticoagulant effect to a medical device or medical material such as a hollow fiber-type dialyzer.

INDUSTRIAL APPLICABILITY

The present invention can be used to impart an excellent anticoagulant effect to a medical device or medical material such as a hollow fiber-type dialyzer.

DESCRIPTION OF SYMBOLS

| | |
|---|---|
| 1a, 1b: | blood port |
| 2a, 2b: | dialysate port |
| 3: | module case |
| 4: | PMMA hollow fiber membrane |
| 5: | potting agent |
| 6: | mini-module |
| 7a, 7b: | silicon tube |
| 8: | peristaltic pump |
| 9: | connecting part |
| 10: | polystyrene round tube |

## Claims

1. A hydrophobic polymer compound, in which a polymer compound inhibiting platelet adhesion is bound with a compound inhibiting blood coagulation reaction.

2. The hydrophobic polymer compound according to claim 1, wherein said polymer compound inhibiting platelet adhesion is a copolymer composed of a hydrophobic polymer and a hydrophilic polymer and adsorbs to polymethyl methacrylate in an amount of not less than 0.1 pg/$mm^2$.

3. The hydrophobic polymer compound according to claim 2, wherein said copolymer is a copolymer of monomers selected from the group consisting of ethylene glycol, vinyl acetate, vinyl chloride, styrene, acrylic acid, acrylate, alkyl acrylate, hydroxyalkyl acrylate, methacrylic acid, methacrylate, alkyl methacrylate, hydroxyalkyl methacrylate, acrylamide, N-alkylamide, N,N-dialkylacrylamide, methacrylamide, N-alkylmethacrylamide, N,N-dialkylmethacrylamide, acrylonitrile, vinyl pyrrolidone, propylene glycol, vinyl alcohol, ethylene, propylene, ethyleneimine, allylamine, vinylamine and siloxane, or a block copolymer composed of said copolymer of said monomers and a polymer selected from the group consisting of polyester, polyamide, polyurethane, polysulfone, polyether sulfone, polycarbonate, polyphenylene sulfide and polyether ether ketone.

**4.** The hydrophobic polymer compound according to claim 2 or 3, wherein said copolymer is a polyether-modified silicone.

**5.** The hydrophobic polymer compound according to any one of claims 1 to 4, wherein said compound inhibiting blood coagulation reaction is a compound having an antithrombin activity.

**6.** The hydrophobic polymer compound according to any one of claims 1 to 5, wherein said compound inhibiting blood coagulation reaction is a compound represented by the following Formula (I):

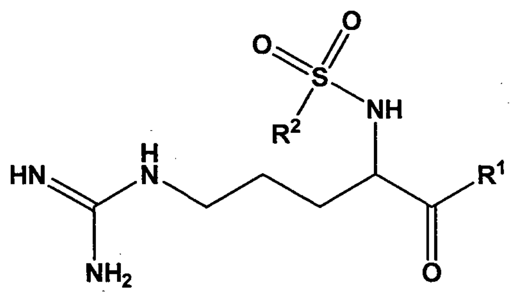

(I)

[wherein, $R^1$ represents a (2R,4R)-4-alkyl-2-carboxypiperidino group; and $R^2$ represents a phenyl group or a fused polycyclic compound group, said fused polycyclic compound group being optionally substituted with a lower alkyl group, a lower alkoxy group or an amino group substituted with a lower alkyl group].

**7.** The hydrophobic polymer compound according to claim 6, wherein said compound represented by said Formula (I) is (2R,4R)-4-methyl-1-((2S)-2-{[(3RS)-3-methyl-1,2,3,4-tetrahydroquinolin-8-yl]sulfonyl}amino-5-guanidinopentanoyl)piperidine-2-carboxylic acid.

**8.** A surface treatment agent of a medical device or medical material, which comprises the hydrophobic polymer compound according to any one of claims 1 to 7 and has an anticoagulant effect.

**9.** A medical device or a medical material, which is treated with the surface treatment agent according to claim 8.

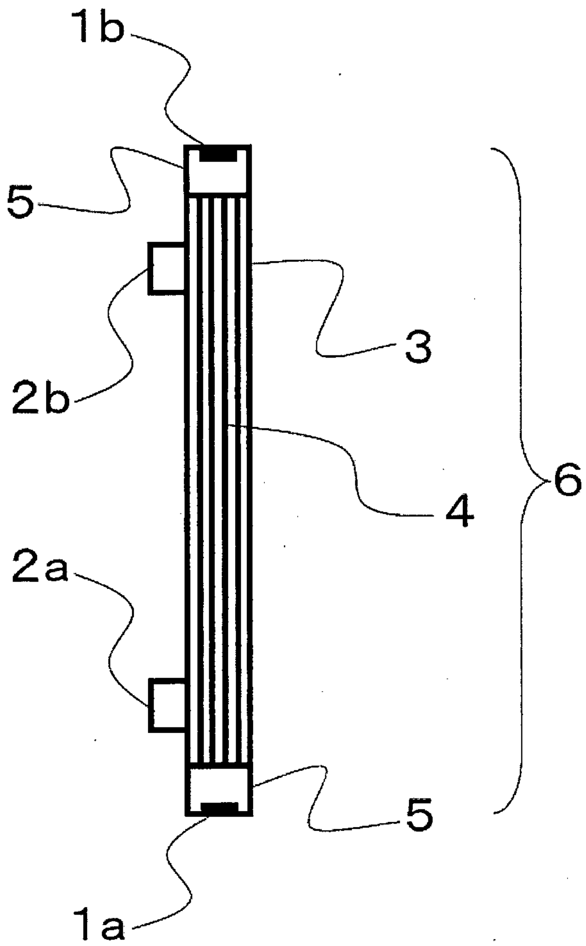


Fig.1

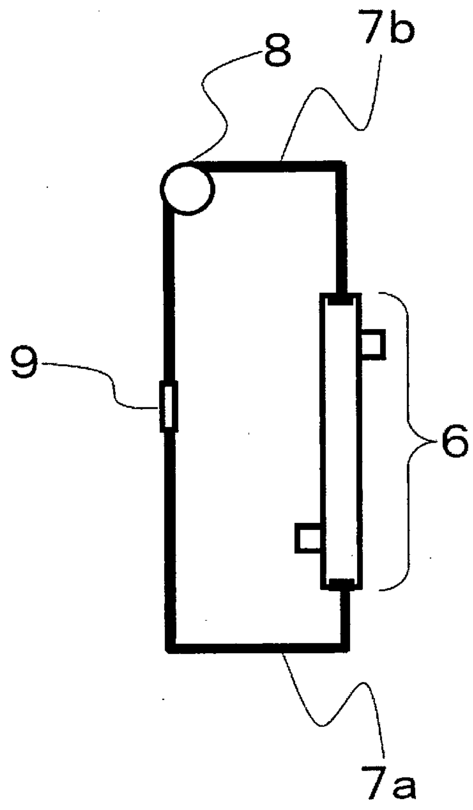


Fig.2

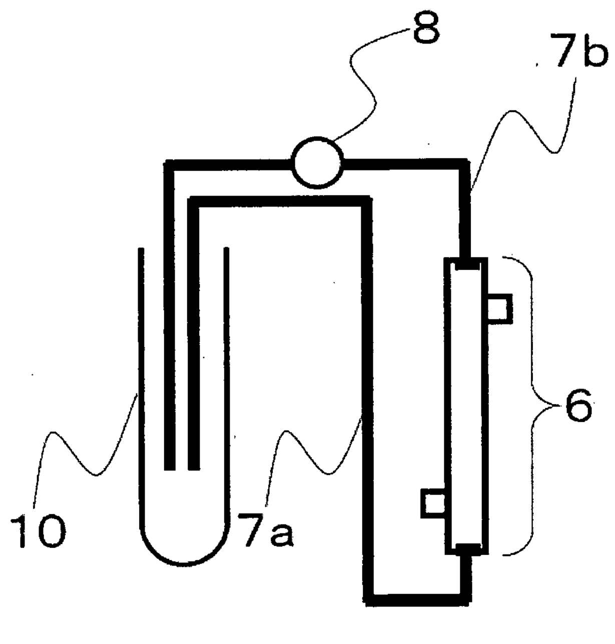


Fig.3

**INTERNATIONAL SEARCH REPORT**

International application No.
PCT/JP2012/065947

A. CLASSIFICATION OF SUBJECT MATTER
*A61L31/00*(2006.01)i, *A61K31/80*(2006.01)i, *A61K47/34*(2006.01)i, *A61K47/48*(2006.01)i, *A61M1/18*(2006.01)i, *A61P7/02*(2006.01)i, *A61K38/55*(2006.01)n

According to International Patent Classification (IPC) or to both national classification and IPC

B. FIELDS SEARCHED

Minimum documentation searched (classification system followed by classification symbols)
A61L31/00, A61K31/80, A61K47/34, A61K47/48, A61M1/18, A61P7/02, A61K38/55

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
CA/REGISTRY/MEDLINE/EMBASE/BIOSIS(STN), JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| X/Y | JP 2003-24452 A (Kawasumi Laboratories, Inc.), 28 January 2003 (28.01.2003), (Family: none) | 1-9/1-9 |
| Y | JP 2005-74139 A (Toto Ltd.), 24 March 2005 (24.03.2005), (Family: none) | 1-9 |
| Y | TSUKAGOSHI Tatsuya. et al, Protein adsorption on polymer-modified silica particle surface Colloids Surf B., 2007, Vol.54 No.1 p.101-107 | 1-9 |
| Y | Akitaka SENUMA, Hiroaki SHOJI, "Silicone aiming at new era. Technological development of modified silicone polymers", JETI., 1995, vol.43, no.12, pages 109 to 111 | 1-9 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 August, 2012 (07.08.12) | 21 August, 2012 (21.08.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2012/065947

C (Continuation). DOCUMENTS CONSIDERED TO BE RELEVANT

| Category* | Citation of document, with indication, where appropriate, of the relevant passages | Relevant to claim No. |
|---|---|---|
| A | WO 2007/145191 A1 (Nippon Shinyaku Co., Ltd.), 21 December 2007 (21.12.2007), & EP 2057985 A1 & US 2010/233262 A1 | 1-9 |
| P,A | WO 2011/078208 A1 (Toray Industries, Inc.), 30 June 2011 (30.06.2011), (Family: none) | 1-9 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2003507082 PCT
- JP 2001213984 A
- JP 2004525888 PCT
- JP 2006291193 A
- WO 08032758 A
- JP 2009225824 A
- JP 2010082067 A
- JP 2007181691 A
- JP 2007181692 A


### Non-patent literature cited in the description


- **MASAMITSU KANAI et al.** Clinical Test Handbook. Kanehara & Co., Ltd, 1993, vol. 30, 416-418